# EUROPÄISCHE PATENTSCHRIFT

(11) **EP 1 743 566 B1**
(45) Veröffentlichungstag und Bekanntmachung des Hinweises auf die Patenterteilung: **18.05.2011**
(21) Anmeldenummer: 06012479.9
(22) Anmeldetag: 19.06.2006
(51) Int. Cl.: A47L 15/23, A61L 2/24

(54) **Spülmaschine**
Rinsing machine
Machine à rincer

(30) Priorität: 15.07.2005 DE 102005033110
(43) Veröffentlichungstag der Anmeldung: 17.01.2007
(73) Patentinhaber: Miele & Cie. KG, 33332 Gütersloh (DE)
(72) Erfinder: Hein, Wolfgang, 33613 Bielefeld (DE); Kethers, Frank, 32791 Lage (DE)

(56) Entgegenhaltungen:
- EP-A2- 0 738 493
- DE-A1- 3 732 451
- DE-A1- 4 020 898
- DE-A1- 10 121 083
- JP-A- 5 031 063

## Beschreibung

Die Erfindung betrifft eine Spülmaschine, insbesondere einen Spülautomat für Labor- und Krankenhausbedarf, mit einem Spülraum, der zur Aufnahme von verschiedenen Spülgutträgern mit rotierbaren Sprüharmen geeignet ist, wobei sich diese Spülgutträger durch die Anzahl und/oder die Höhenposition ihrer Sprüharme unterscheiden.

Eine Spülmaschine dieser Art wird von der Anmelderin als Reinigungs- und Desinfektionsgerät G 7824 Desinfektor vertrieben und ist beispielsweise aus dem Prospekt "Miele Professional ― Systemlösungen für die ZSVA im Krankenhaus" bekannt. Bei einem solchen Gerät werden als Spülgutträger Einsatzwagen verwendet, die für den jeweiligen Anwendungszweck bzw. das aufzunehmende Spülgut angepasst sind. Dabei können zur Spülflüssigkeitsverteilung an den Einsatzwagen rotierende Sprüharme in unterschiedlicher Anzahl und Höhe angeordnet sein. Außerdem ist in der G 7824 Desinfektor selbst je ein oberer und unterer Maschinensprüharm fest installiert.

Es besteht der Wunsch zu überwachen, ob sich alle Sprüharme korrekt drehen oder ob der eine oder andere Sprüharm beispielsweise durch falsch abgelegtes Spülgut blockiert ist und deshalb das Spülergebnis verschlechtert wird. Es ist bei Haushaltsgeschirrspülmaschinen allgemein bekannt, die Rotation des Korbsprüharms zu kontrollieren. Dabei kommen Mikrofone (DE 40 10 066 C2), Magnetsensoren (DE 37 32 451 C2) oder schaltende Wippen (DE 32 33 501 C1) zum Einsatz. In der DE 37 32 451 C2 wird der Verstellbarkeit des Oberkorbes und der sich dadurch ändernden Höhenlage des Sprüharms durch die Anordnung eines Jochs vor dem Magnetsensor Rechnung getragen. Mit der gezeigten Anordnung ist es aber nicht möglich, die Rotation bei verschiedenen Spülgutträgern mit Sprüharmen in unterschiedlicher Anzahl und Höhe korrekt zu überwachen. Aus der DE 40 10 066 C2 ist es bekannt, durch Signalfilterung mit einem einzigen Sensor mehrere Sprüharme gleichzeitig zu überwachen. Dies ist jedoch nur bei einem unveränderlichen System möglich, bei dem die Sprüharme unveränderte Signale abgeben. Eine Erkennung von Variationen aufgrund unterschiedlicher Sprüharmanordnungen ist nicht möglich.

Aus der EP 738 493 A2 ist es bekannt, die Einsatzwagen einer Spülmaschine mit einem Magnetschlüssel auszustatten, der von der Maschine erkannt wird. Durch den Magnetschlüssel werden die Reinigungsprogramme an den jeweiligen Wagen und dadurch an das darauf befindliche Spülgut angepasst.

Der Erfindung liegt deshalb das Problem zugrunde, bei einer Spülmaschine der eingangs genannten Art trotz des Einsatzes von Spülgutträgern mit Sprüharmen in unterschiedlicher Anzahl und/oder Höhenposition eine Rotationsüberwachung der Sprüharme zu ermöglichen.

Erfindungsgemäß wird dieses Problem durch eine Spülmaschine mit den Merkmalen des Patentanspruchs 1 gelöst. Vorteilhafte Ausgestaltungen und Weiterbildungen der Erfindung ergeben sich aus den nachfolgenden Unteransprüchen.

Die mit der Erfindung erreichbaren Vorteile ergeben sich aus der Möglichkeit, der Auswerteschaltung die Information mitzugeben, aus der sie erkennt, bei welchen Sensoren aufgrund der individuellen Höhenpositionen der Sprüharme des im Spülraum befindlichen Spülgutträger Signale zu erwarten sind. Hierdurch werden bei jedem Einsatzwagen nur die Sprüharme auf Rotation überwacht, die tatsächlich vorhanden sind und es kann kein Fehlsignal durch die ausbleibende Rotation eines gar nicht vorhandenen Sprüharms erzeugt werden.

Dabei ist es zweckmäßig, wenn die Sensoren in bekannter Weise als Magnetsensoren ausgebildet sind und wenn in jedem Sprüharm ein Dauermagnet angeordnet ist. In einer vorteilhaften Ausführungsform erfassen die Sensoren außerdem jeweils einen räumlich ausgedehnten Sensorbereich. Hierdurch ist es möglich, geringfügige Höhenunterschiede, etwa bedingt durch neue Gestaltungsvarianten, zu kompensieren. Dabei ist es auch zweckmäßig wenn sich die Sensorbereiche überlappen.

In einer weiteren vorteilhaften Ausführungsform ist die Eingabeeinrichtung als System aus einem am Spülgutträger angeordneten Magnetschlüssel und einer am Spülraum angeordneten Magnetsensor-Leiste ausgebildet. Hierdurch wird der Bedienkomfort erhöht. Zwar ist die Eingabe der notwendigen Information über die Anzahl und Höhenposition der Sprüharme auch durch Bedientasten, Magnetkarten o. ä. möglich, dabei besteht jedoch die Möglichkeit von Fehleingaben, die zu unerwünschten Programmunterbrechungen oder falschen Warnanzeigen führen können. Alternativ kann die Eingabeeinrichtung als System aus einem am Spülgutträger angeordneten Transponder und einer am Spülraum angeordneten Transponder-Leseeinrichtung ausgebildet sein.

Sollte es aufgrund geringer Abstände der einzelnen Sprüharme zu Problemen hinsichtlich der Erkennung der einzelnen Dauermagnete kommen, da sich deren Magnetfelder zu stark überschneiden, so kann alternativ jeder Sprüharm mit einem Transponder ausgestattet sein, wobei sich die einzelnen Transponder hinsichtlich eines abgespeicherten Identifikationscodes unterscheiden, und die Sensoren sind als Antennen zum Empfang der von den Transpondern abgestrahlten Identifikationscodes ausgebildet.

Um den Benutzer auf eine Blockierung eines oder mehrerer Sprüharme hinzuweisen ist es vorteilhaft, wenn eine Warnanzeige vorgesehen ist, welche durch die Auswerteschaltung bei Unterbleiben eines erwarteten Sensorsignals aktivierbar ist. Alternativ kann ein Programmabbruch erfolgen.

Ein Ausführungsbeispiel der Erfindung ist in den Zeichnungen rein schematisch dargestellt und wird nachfolgend näher beschrieben. Es zeigt
- Figur 1: einen mit einem Einsatzwagen für Spülgut beladenen Spülautomaten mit geöffnetem Spülraum in der Seitenansicht;
- Figur 2a bis c: rein schematisch den Spülraum mit Rotationssensoren und unterschiedlichen Einsatzwagen;

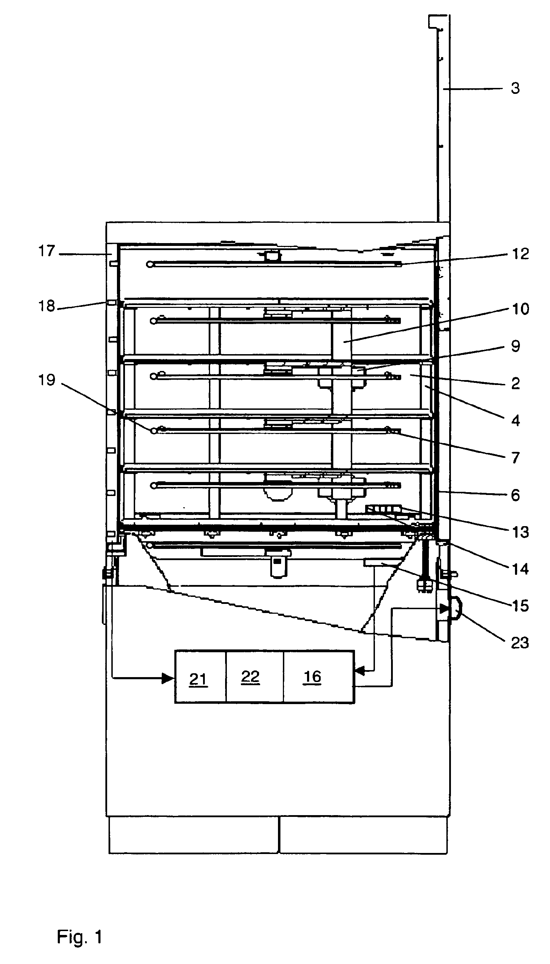

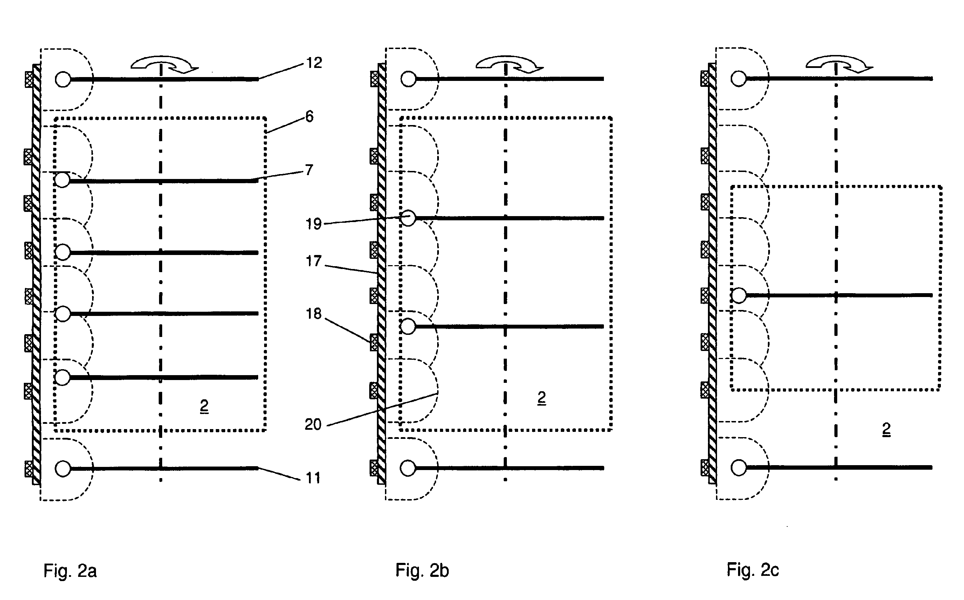

Figur 1 zeigt einen Spülautomat 1, der im Labor- und Krankenhausbereich zum programmgesteuerten Reinigen, Desinfizieren und Trocknen von Spülgut, wie Atemschläuche, Katheter, OP-Instrumente, Pipetten oder dergl. eingesetzt wird. Er besitzt einen Spülraum 2, welcher in bekannter Weise durch eine Gerätetür - im Ausführungsbeispiel eine vertikal verschiebbare Hubtür 3 ― verschließbar ist. Das Spülgut (nicht dargestellt) wird in Körben 4 abgelegt, welche wiederum in einem Einsatzwagen 6 in den Spülraum 2 eingeschoben werden, dabei werden je nach Art, Größe und Menge des Spülguts unterschiedliche Wagen 6 verwendet. Diese Wagen 6 sind mit rotierbaren Sprüharmen 7 ausgestattet, deren Anzahl und Anordnung bzgl. ihrer Höhenposition verschieden sein kann. Hier dient die schematische Darstellung in Figur 2a bis c von drei Einsatzwagen 6 mit vier Sprüharmen 7, zwei Sprüharmen 7 und einem Sprüharm 7 als beispielhafte, aber nicht abschließende Aufzählung. Die einzelnen Sprüharme 7 werden in bekannter Weise über ein Adapterstück 9 an die Spülflüssigkeitszufuhr 10 angekuppelt. Durch den seitlichen Austritt der Spülflüssigkeit aus den Antriebsdüsen (nicht dargestellt) werden die Sprüharme nach dem Jetprinzip in Rotation versetzt. Zusätzlich zu den Sprüharmen 7 am Einsatzwagen ist ein unterer 11 und ein oberer 12 Maschinensprüharm fest am Spülraum installiert.

Für die Behandlung oder Aufbereitung des Spülgutes im Spülautomaten 1 stehen mehrere separat aufrufbare Reinigungs- und Desinfektionsprogramme zur Verfügung, welche spülgut- bzw. einsatzwagenabhängig auswählbar sind. Entsprechend sind die unterschiedlichen Einsatzwagentypen notwendig. Hierfür ist, wie aus der EP 0 738 493 A2 bekannt, jeder Einsatzwagen 6 mit einem Magnetschlüssel 13 aus verschieden gepolten Einzelmagneten 14 versehen. Die Magnetisierungsrichtung dieser Einzelmagnete 14 wird von einer unter dem Spülraum angeordneten Magnetsensor-Leiste 15 erkannt und von der Gerätesteuerung 16 in ein entsprechendes Spülprogramm für das im Korb 4 geladene Spülgut umgesetzt.

Zur Erkennung der Sprüharmrotation sind an einer der Seitenwände 17 des Spülraums 2 mehrere Magnetsensoren 18, beispielsweise magnetoresistive Sensoren mit entsprechender Signalaufbereitung eingesetzt. Außerdem ist jeder Sprüharm 7, 11 und 12 an einem Ende mit einem Dauermagneten 19 ausgestattet. Die Magnetsensoren 18 erzeugen ein Signal, wenn das mit einem Dauermagnet 19 besetzte Ende eines Sprüharms 7, 11 und 12 infolge seiner Rotation sich durch den mit gestricheltem Halbkreis 20 angedeuteten Erfassungsbereich des Sensors 18 bewegt. Die Signale aller Sensoren 18 werden an eine in der Gerätesteuerung 16 integrierte Auswerteschaltung 21 weitergeleitet und diese erkennt aus dem Signalverlauf des jeweiligen Sensors 18 die ordnungsgemäße Rotation des Sprüharms 7, 11 und 12 in seinem Erfassungsbereich 20. Um die Anzahl der Magnetsensoren 18 trotz fertigungs- oder änderungsbedingter Höhendifferenzen in den möglichen Sprüharmpositionen gering zu halten, erfassen die Sensoren 18 jeweils einen räumlich ausgedehnten Sensorbereich 20 und die Sensorbereiche 20 überlappen sich.

Aufgrund der unterschiedlichen Sprüharm-Ausstattung der Einsatzwagen 6 benötigt die Auswerteschaltung 21 eine Information darüber, von welchen Sensoren 18 überhaupt Signale zu erwarten sind. Andernfalls würde sie auch von einem Sensor 18, in dessen Höhenposition gar kein Sprüharm 7 vorhanden ist, ein Signal erwarten und dessen Ausbleiben als Sprüharm-Blockade auswerten. Die entsprechende Information erhält die Auswerteschaltung 21 durch den Magnetschlüssel 13 am jeweiligen Einsatzwagen 6. Entsprechend dem Code, der durch den Magnetschlüssel 13 über die Magnetsensor-Leiste 15 an die Gerätesteuerung 16 weitergegeben wird, liest diese aus einer Tabelle aus einem nichtflüchtigen Speicher 22 eine Information über die individuellen Höhenpositionen der einzelnen Sprüharme aus, und gibt sie an die Auswerteschaltung 21 weiter. Die Auswerteschaltung 21 überwacht dann nur die Sensoren 18, von denen bei ordnungsgemäßer Rotation ein Signal zu erwarten ist. Wenn dieses Signal unterbleibt, schließt sie auf eine Sprüharm-Blockade und aktiviert eine Warnanzeige 23 oder unterbricht den Programmablauf des Spülautomaten 1.

Anstelle des Magnetschlüssels 13 und der Magnetsensor-Leiste 15 kann ein in den Zeichnungen nicht dargestelltes System aus einem am Spülgutträger angeordneten Transponder und einer am Spülraum angeordneten Transponder-Leseeinrichtung verwendet werden.

Bei Einsatzwagen mit einer großen Anzahl von Sprüharmen, d.h. kleinen Abständen zwischen den einzelnen Armen, kann es zu einer starken Überschneidung der Magnetfelder der Dauermagneten kommen. Bei einem solchen, in den Zeichnungen nicht dargestellten Spülautomat kann dann alternativ jeder Sprüharm mit einem Transponder ausgestattet sein, wobei sich die einzelnen Transponder hinsichtlich eines abgespeicherten Identifikationscodes unterscheiden. Außerhalb des Spülraums müssen dann geeignete Antennen angeordnet sein, die die Transpondersignale nur beim Vorbeistreichen des Transponders empfangen. Dreht sich ein Sprüharm und erreicht das Wirkfeld der Antenne, so wird der im Sprüharm angeordnete Transponder ausgelesen und gibt seinen Identifikationscode an die Auswerteschaltung weiter. Diese erkennt beim Ausbleiben des Signals die Sprüharmblockade.

Für die Entscheidungsfindung, ob sich die richtige Anzahl von Sprüharmen dreht, muss auch hier die Auswerteschaltung wissen, welche Identifikationscodes zu erwarten sind. Diese Information kann durch eine der vorbeschriebenen Vorrichtungen (Magnetschlüssel oder Transponder) sichergestellt werden.

## Patentansprüche

1. Spülmaschine, insbesondere Spülautomat (1) für Labor- und Krankenhausbedarf, mit einem Spülraum (2), der zur Aufnahme von verschiedenen Spülgutträgern (4, 6) mit rotierbaren Sprüharmen (7) geeignet ist, wobei sich diese Spülgutträger (4, 6) durch die Anzahl und/oder die Höhenposition ihrer Sprüharme (7) unterscheiden, mit im Bereich einer Spülraumwand (17) angeordneten Sensoren, welche den unterschiedlichen Höhenpositionen der Sprüharme (7) zugeordnet sind und welche in der Lage sind, ein Signal abzugeben, wenn ein rotierender Sprüharm (7) in ihren Erfassungsbereich (20) tritt, mit einer Auswerteschaltung (21), welche mit Hilfe des Signals die ordnungsgemäße Rotation des Sprüharms (7) überwacht, und mit einer Einrichtung zur Eingabe einer Information, aus der die Auswerteschaltung erkennt (21), bei welchen Sensoren aufgrund der individuellen Höhenpositionen der Sprüharme (7) des im Spülraum (2) befindlichen Spülgutträgers (4, 6) ein Signal zu erwarten ist.

2. Spülmaschine nach Anspruch 1,
**dadurch gekennzeichnet,**
**dass** die Sensoren als Magnetsensoren (18) ausgebildet sind und dass in jedem Sprüharm (7) mindestens ein Dauermagnet (19) angeordnet ist.

3. Spülmaschine nach Anspruch 2,
**dadurch gekennzeichnet,**
**dass** die Sensoren (18) jeweils einen räumlich ausgedehnten Sensorbereich (20) erfassen.

4. Spülmaschine nach Anspruch 3,
**dadurch gekennzeichnet,**
**dass** sich die Sensorbereiche (20) überlappen.

5. Spülmaschine nach einem der Ansprüche 1 bis 4,
**dadurch gekennzeichnet,**
**dass** die Sensoren den Innenraum über seine gesamte Höhe abtasten.

6. Spülmaschine nach einem der Ansprüche 1 bis 5,
**dadurch gekennzeichnet,**
**dass** die Eingabeeinrichtung als System aus einem am Spülgutträger (4, 6) angeordneten Magnetschlüssel (13) und einer am Spülraum (2) angeordneten Magnetsensor-Leiste (15) ausgebildet ist.

7. Spülmaschine nach einem der Ansprüche 1 bis 5,
**dadurch gekennzeichnet,**
**dass** die Eingabeeinrichtung als System aus einem am Spülgutträger (4, 6) angeordneten Transponder und einer am Spülraum (2) angeordneten Transponder-Leseeinrichtung ausgebildet ist.

8. Spülmaschine nach einem der Ansprüche 6 oder 7,
**dadurch gekennzeichnet,**
**dass** der Auswerteschaltung (21) ein Speicher (22) zugeordnet ist, in welchem von der Eingabeeinrichtung zu erwartende Codes und hierzu die Sensoren (18), von denen Signale zu erwarten sind, abgelegt sind.

9. Spülmaschine nach Anspruch 8,
**dadurch gekennzeichnet,**
**dass** die Codes und die zugehörigen Sensoren (18) in Form einer Tabelle abgelegt sind.

10. Spülmaschine nach Anspruch 1,
**dadurch gekennzeichnet,**
**dass** jeder Sprüharm mit einem Transponder ausgestattet ist, wobei sich die einzelnen Transponder hinsichtlich eines abgespeicherten Identifikationscodes unterscheiden, und dass die Sensoren als Antennen zum Empfang der von den Transpondern abgestrahlten Identifikationscodes ausgebildet sind.

11. Spülmaschine nach einem der Ansprüche 1 bis 10,
**dadurch gekennzeichnet,**
**dass** eine Warnanzeige (23) vorgesehen ist, welche durch die Auswerteschaltung (21) bei Unterbleiben eines erwarteten Sensorsignals aktivierbar ist.

## Claims

1. Rinsing machine, in particular an automatic rinsing machine (1) for use in laboratories and hospitals, comprising a rinsing chamber (2) which is adapted to receive different carriers (4, 6) for articles to be rinsed, said carriers (4, 6) having rotatable spray arms (7) and differing from one another in the number and/or vertical position of their spray arms (7), comprising sensors which are arranged in the region of a rinsing chamber wall (17), associated with the different vertical positions of the spray arms (7) and capable of emitting a signal when a rotatable spray arm (7) enters its detection region (20), comprising an evaluation circuit (21) which uses the signal to monitor the proper rotation of the spray arm (7), and comprising a device for inputting information from which the evaluation circuit (21) determines those sensors from which a signal is to be expected, based on the individual vertical positions of the spray arms (7) of the carriers (4, 6) for articles to be rinsed located in the rinsing chamber (2).

2. Rinsing machine according to claim 1,
**characterised in that**
the sensors are formed as magnetic sensors (18) and **in that** at least one permanent magnet (19) is arranged in each spray arm (7).

3. Rinsing machine according to claim 2,
**characterised in that**
the sensors (18) detect a spatially extensive sensor region (20) in each case.

4. Rinsing machine according to claim 3,
**characterised in that**
the sensor regions (2) overlap one another.

5. Rinsing machine according to any one of claims 1 to 4,
**characterised in that**
the sensors scan the interior over the entire height thereof.

6. Rinsing machine according to any one of claims 1 to 5,
**characterised in that**
the input device is formed as a system comprising a magnetic key (13) arranged on the carrier (4, 6) for articles to be rinsed and a magnetic sensor strip (15) arranged on the rinsing chamber (2).

7. Rinsing machine according to any one of claims 1 to 5,
**characterised in that**
the input device is formed as a system comprising a transponder arranged on the carrier (4, 6) for articles to be rinsed and a transponder reading device arranged on the rinsing chamber (2).

8. Rinsing machine according to either claim 6 or claim 7,
**characterised in that**
a storage means (22) is associated with the evaluation circuit (21), in which storage means codes to be expected from the input means, and for this purpose the sensors (18) from which signals are to be expected, are stored.

9. Rinsing machine according to claim 8,
**characterised in that**
the codes and the associated sensors (18) are stored in the form of a table.

10. Rinsing machine according to claim 1,
**characterised in that**
each spray arm is equipped with a transponder, the individual transponders differing from one another in terms of a stored identification code, and **in that** the sensors are formed as antennae for receiving the identification codes emitted by the transponders.

11. Rinsing machine according to any one of claims 1 to 10,
**characterised in that**
a warning display (23) is provided which can be activated by the evaluation circuit (21) when an expected sensor signal does not occur.

## Revendications

1. Machine à rincer, en particulier machine à rincer automatique (1) pour les besoins des laboratoires et des hôpitaux, avec une chambre de rinçage (2) qui est adaptée pour recevoir divers supports de produits à rincer (4, 6) avec des bras de rinçage (7), ces supports de produits à rincer (4, 6) se différenciant par le nombre et/ou la position en hauteur de leurs bras de rinçage (7), avec des capteurs disposés dans la zone d'une paroi de chambre de rinçage (17) qui sont affectés aux différentes positions en hauteur des bras de rinçage (7) et qui sont en mesure de délivrer un signal quand un bras de rinçage (7) tournant entre dans leur zone de détection (20), avec un circuit d'analyse (21) qui surveille à l'aide du signal la rotation conforme du bras de rinçage (7), et avec un équipement pour l'entrée d'une information à partir de laquelle le circuit d'analyse (21) détecte de quels capteurs il faut attendre un signal du fait des positions en hauteur individuelles des bras de rinçage (7) du support de produits à rincer (4, 6) situé dans la chambre de rinçage (2).

2. Machine à rincer selon la revendication 1,
**caractérisée en ce que**
les capteurs sont constitués en tant que capteurs magnétiques (18) et **en ce que**, dans chaque bras de rinçage (7), il est disposé au moins un aimant permanent (19).

3. Machine à rincer selon la revendication 2,
**caractérisée en ce que**
les capteurs (18) détectent respectivement une zone de capteur (20) étendue dans l'espace.

4. Machine à rincer selon la revendication 3,
**caractérisée en ce que**
les zones de capteur (20) se chevauchent.

5. Machine à rincer selon l'une des revendications 1 à 4,
**caractérisée en ce que**
les capteurs explorent la chambre intérieure sur toute sa hauteur.

6. Machine à rincer selon l'une des revendications 1 à 5,
**caractérisée en ce que**
l'équipement d'entrée est constitué, en tant que système, d'une clé magnétique (13) disposée sur le support de produits à rincer (4, 6) et d'une barrette de capteurs magnétiques (15) disposée sur la chambre de rinçage (2).

7. Machine à rincer selon l'une des revendications 1 à 5,
**caractérisée en ce que**
l'équipement d'entrée est constitué, en tant que système, d'un transpondeur disposé sur le support de produits à rincer (4, 6) et d'un équipement de lecture pour transpondeur disposé sur la chambre de rinçage (2).

8. Machine à rincer selon l'une des revendications 6 ou 7,
**caractérisée en ce que**,
au circuit d'analyse (21), il est affecté une mémoire (22) dans laquelle sont déposés des codes que l'on doit attendre de l'équipement d'entrée et, pour cela, les capteurs (18) dont on doit attendre des signaux.

9. Machine à rincer selon la revendication 8,
**caractérisée en ce que**
les codes et les capteurs (18) correspondants sont déposés sous forme de tableau.

10. Machine à rincer selon la revendication 1,
**caractérisée en ce que**
chaque bras de rinçage est équipé d'un transpondeur, les différents transpondeurs se différenciant en ce qui concerne un code d'identification enregistré, et **en ce que** les capteurs sont constituées en tant qu'antennes pour la réception des codes d'identification émis par les transpondeurs.

11. Machine à rincer selon l'une des revendications 1 à 10,
**caractérisée en ce**
**qu'**il est prévu une annonce d'alarme (23) qui peut être activée par le circuit d'analyse (21) en cas d'absence d'un signal de capteur attendu.
